# EUROPEAN PATENT APPLICATION

(11) **EP 3 679 776 A1**
(43) Date of publication of application: **15.07.2020**
(21) Application number: 19151420.7
(22) Date of filing: 11.01.2019
(51) Int. Cl.: A01B 79/00, A01C 21/00

(54) **METHOD OF COLLECTING SOIL DATA VIA AN UAV**

(71) Applicant: GE Aviation Systems Limited, Bishops Cleeve Cheltenham Gloucestershire GL52 8SF (GB)
(72) Inventor: SCHWINDT, Stefan, Cheltenham, Gloucestershire GL52 8SF (GB)
(74) Representative: Openshaw & Co.

(57) **Abstract**

A method 100 of determining soil condition for possible treatment. The method 100 comprises taking a soil sample 101 at a predetermined location with an unmanned aerial vehicle (UAV) 10. Sensing 102 at least one characteristic of the soil with a sensor 26. Storing a value 103 representative of the at least one characteristic and the predetermined location in a database. Determining 104 if the value is within an acceptable range, and taking a corrective action 105 to adjust the valve within the acceptable range.

## Description

### BACKGROUND

In contemporary farming, monitoring soil conditions on farm land is vital to maximizing crop yields. Visual inspections and soil sampling can help a farmer or land owner determine soil conditions and whether applications of fertilizer, herbicide or insecticide would be beneficial for crop growth and optimization. If soil characteristics are not optimized, crop yields and optimization can suffer. In addition, visual inspection could be performed by government of regulatory bodies to monitor environmental compliance.

### BRIEF DESCRIPTION

In one aspect, the present disclosure relates to a method of determining soil condition for possible treatment. The method comprises taking a soil sample at a predetermined location with an unmanned aerial vehicle (UAV). Sensing, at least one characteristic of the soil with a sensor. Storing a value representative of the at least one characteristic and the predetermined location in a database. Determining if the value is within an acceptable range, and taking a corrective action to adjust the valve within the acceptable range.

In another aspect, the present disclosure relates to a method of determining soil condition for possible treatment. The method comprises taking a soil sample at a preprogrammed GPS location with an unmanned aerial vehicle (UAV). Sensing, with a sensor, a characteristic of a resource applied to the soil. Storing a value representative of the characteristic and the GPS location in a database. Determining if the value is within an acceptable range, and taking a corrective action to adjust the valve within the acceptable range.

### BRIEF DESCRIPTION OF THE DRAWINGS

In the drawings:
FIG. 1 is a schematic illustration of an unmanned aerial vehicle (UAV) for land observation according to aspects of the present disclosure as described herein.
FIG. 2 is a schematic illustration of an exemplary an unmanned aerial vehicle (UAV) for land observation of FIG. 1 with the addition of a ground system and associated electronic equipment.
FIG. 3 is a flow chart illustrating a method of determining soil condition for possible treatment.

### DETAILED DESCRIPTION

Aspects of the present disclosure speak to a land monitoring system and a method of collecting soil sampling data with an unmanned aerial vehicle (UAV). The land monitoring system, specifically for use on farm lands, can include a UAV having one or more tools, one or more sensors, one or more cameras and the capability to communicate with a ground station in communication with a computer system configured to store, manipulate and analyze the soil sample data. Based on the analysis of the data, the system can take corrective actions, or alert the land owner to take corrective actions.

As used herein, "a set" can include any number of the respectively described elements, including only one element. All directional references (e.g., radial, axial, proximal, distal, upper, lower, upward, downward, left, right, lateral, front, back, top, bottom, above, below, vertical, horizontal, clockwise, counterclockwise, upstream, downstream, forward, aft, etc.) are only used for identification purposes to aid the reader's understanding of the present disclosure, and do not create limitations, particularly as to the position, orientation, or use of the disclosure. Connection references (e.g., attached, coupled, connected, and joined) are to be construed broadly and can include intermediate members between a collection of elements and relative movement between elements unless otherwise indicated. As such, connection references do not necessarily infer that two elements are directly connected and in fixed relation to one another. The exemplary drawings are for purposes of illustration only and the dimensions, positions, order, and relative sizes reflected in the drawings attached hereto can vary.

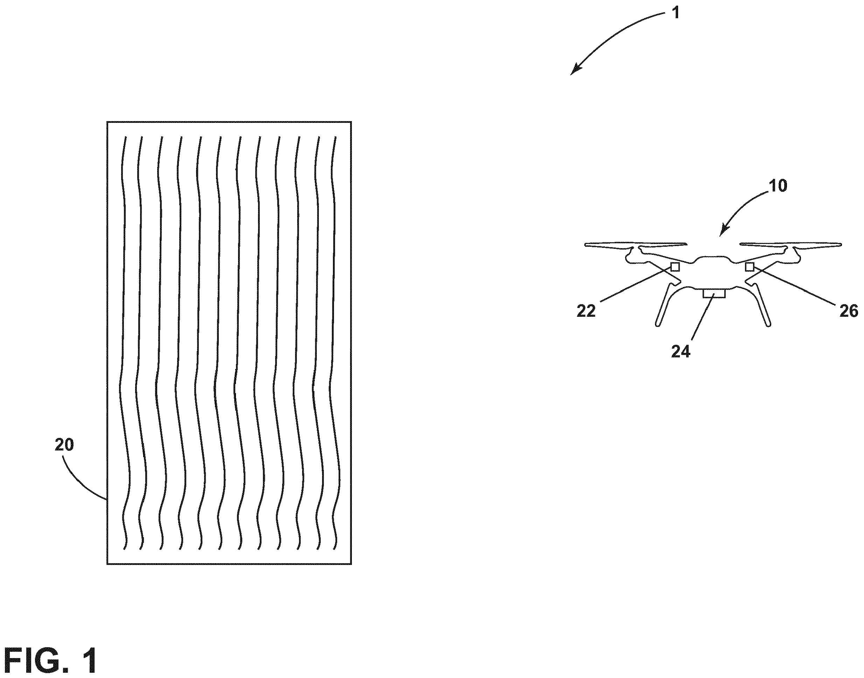

FIG. 1 illustrates an exemplary system 1 of providing an unmanned aerial vehicle (UAV) 10 for land 20 observation. More specifically, the system 1 can be used to monitor land, such as farm land, and to take soil or vegetation samples of the farm land 20 for the purpose of detecting soil characteristics such as moisture content, fertilizer, insecticide, herbicide, or other chemical agents. The UAV 10 can be provided with one or more cameras 22 for machine vision, one or more tools 24 for collecting soil samples, and or one or more sensors 26 such as a spectroscope, a mass spectrometer, a gas chromatograph, or a combination thereof that can be used to identify soil characteristics. The UAV 10 can be configured to fly over farm land 20 and capture visual images of the land and collect and analyze soil samples at various locations on the farm land 20 for soil characteristic analysis. For example, a combined gas chromatograph-mass spectrometer can vaporize a soil sample with a laser, and the gas chromatograph can separate it into separate molecules of different sizes and the mass spectrometer can identify each molecule the constituent atoms/isotopes

In more detail, the UAV 10 can be programmed or controlled to fly over all or portions of land 20 to capture images of the land 20. Due the versatile, precise, and controllable flying nature of typical UAV's 10, images of land 20 can be taken from high elevational views to capture overall aerial views of the land 20 or can be taken at very low levels, even as closes a 1-2ft, for capturing close up images of land 20. The use of GPS can allow for precise knowledge of where photos are taken and from identified sensors angles. Thus, it can be easy to accurately stitch images together or to identify a location of a close up image within the overall aerial view. For example, the UAV could programmed to fly up to a high altitude to take a picture of whole field, then programmed to take sampling photos at a lower level in a defined range (e.g. 1m x 1m land plots). To accomplish this, the UAV 10 can be provided with different types of camera lenses for close up images, wide-angle images, infrared images, and others. The UAV 10 can be provided with a combination of lenses and flying instructions to capture close up images of soil including weeds, fungus or other vegetation that may be visible on top of the soil, moisture content based on the color of the soil, or high level views to capture the layout or look of the land. The images captured by the camera or cameras 22 can be stored as data and can be transmitted to a ground station or computer for analysis.

The UAV 10 can also outfitted with one or more instruments or tools 24 capable of penetrating the soil and collecting soil or vegetation samples. In some embodiments, the tools 24 can be configured to collect top soil or loose vegetation with scoops or claws, or can be configured with tools capable of drilling or digging into the farm land 20 for extracting soil at a depth below ground level. In an exemplary embodiment, the tools 24 can allow the UAV to collect samples of at least 6 inches to a couple feet below the land surface 20 to allow for analysis of penetration of moisture or chemicals such as fertilizers or insecticides into the land 20. The UAV 10 can programmed or controlled to collect soil or vegetation samples at various locations across the land and at various depths. For example, in some locations it might be beneficial to collect top soil or vegetation for analysis and in other locations it might be beneficial collect a deeper soil sample to determine chemical penetration.

In an alternate embodiment, the UAV 10 could be outfitted with tools capable of cutting or pulling vegetation. In one example, if the UAV 10 identifies an invasive vegetation such as weed, the UAV could be programmed to identify such vegetation and cut or pull the vegetation.

The UAV 10 can also be provided with one or more sensors 26 such as a spectroscope, mass spectrometer, or a gas chromatograph that can be used to identify soil composition or characteristics. The one or more sensors 26 can be in communication with the one or more tools 24 for analyzing the collected the soil or vegetation samples. The sensors 24 can be programmed or capable of sensing one or more characteristics of the soil including moisture content, or the presence or concentration of chemicals, fertilizers, herbicides, or insecticides. In addition, the camera 22 can be used to identify both the need for fertilizers, herbicides or insecticides (i.e. if there are no weeds, there is no need for a herbicide application) as well as the efficiency of a fertilize, insecticide, or herbicide application. For example, if a herbicide application has been applied, the camera may take one or more images of the vegetation over a period of time and this data can be used to determine if another application is necessary.

It should be recognized that sensors 24 can come in different shapes, sizes and weights and not all UAV's 10 will be of the size or have the flying power to carry the one or more sensors 26. In the case where the UAV is not capable of carrying a larger sensor 26 such as spectroscope, mass spectrometer or gas chromatograph, these sensors 26 could be maintained at a ground station, or a moving ground station such as a tractor. The ground station can be configured to carry any sensors 26 required for soil or vegetation analysis. Values indicative of the characteristics of the soil, the depth at which the soil sample was taken, the GPS coordinates of where the soil sample was taken and other data can be stored in the UAV 10 or in a computer memory in a ground station.

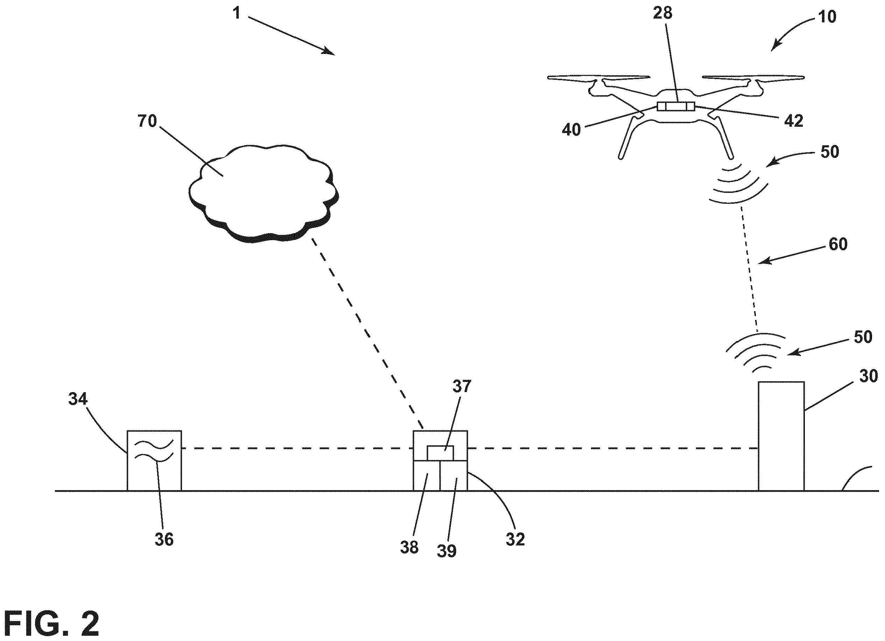

FIG. 2 shows the exemplary system 1 with the addition of a ground system 30 and associated electronic equipment. As used herein the term ground system or ground station means a computer terminal linked to the UAV 10 by an antenna and including any associated electronic equipment for the purpose of transmitting or receiving messages, tracking, or controlling or the UAV 10. It should be recognized that a ground station 30 can be fixed such as situated in a barn or house, or could be mobile such as attached to tractor or other moving object.

In this illustration, the UAV 10 can be configured to carry or otherwise house a communication device 28. The communication device 28 can be configured for at least one-way communication of data from the UAV 10 to the ground station 30. It is also contemplated that the communication device 28 can be a transceiver capable of two-way communication between the UAV 10 and the ground station 30. It is further contemplated that the communication device 8 can be WiFi or Bluetooth enabled or enabled the any other wired or wireless standard communication protocol. The communication device 28 further includes at least one of a memory 40 and a controller 42 that are operably coupled to the communication device 28 for sending and receiving data. Data as used herein can mean any information collected, received or stored in the memory 40 of the UAV 10 such as images collected by the one or more cameras 22, soil characteristics analyzed by the sensors 26, GPS coordinates, soil sample depth coordinates, flying instructions, or any other data transmitted, received, measured, or analyzed by the UAV 10..

The UAV 10 can be deployed and controlled by preprogramming a computer 32 in communication with the ground station 30 with flight path instructions and samples to be collected from various locations on the land 20. For example, the computer 32 could programmed with instructions that send the UAV 10 to various location, take various pictures of the land 20 at predetermined heights, and collect soil samples at one or more depths from various locations. Alternatively, the UAV 10 can be manually deployed and controlled. For example, an authorized individual such as a farmer, land developer or government official could manually deploy and control the UAV 10 to various locations on the land and could manually control image taking with cameras 22 or manually control the depth and extent of soil or vegetation collection on the land 20. It is further contemplated that the deployment or the control of the UAV 10 can include both automatic computer control and manual deployment.

Additionally, the UAV 10 could be controlled by artificial intelligence where the UAV 10 is not preprogrammed to specific flights or tests or manually flown by human, but is programmed with intelligence to allow the UAV 10 to make judgments about operations. For example, the UAV 10 could fly up to take an overall aerial picture based on data provided as to the boundaries of the farm land 20. The UAV 10 could use the data to establish a perimeter and place a form of grid to optimize sample collection. The UAV 10 could sample soil in a determined radius, and the UAV 10 could then work out optimal flight path and number of samples to cover the entire ground. It could also use the aerial photo to identify areas of greater or lesser interest such as vegetation density, rivers, man mad structures, etc. In areas where values raise a concern, the UAV 10 could collect a higher numbers of samples and determine appropriate application or non-application of water, herbicides, fertilizers or insecticides.

A communication link 50 can be establish between the UAV 10 and the ground station 30 for transmitting data 60 between the UAV and ground station 30. By way of non-limiting example, the controller 42 can initiate the communication device 28 on the UAV 10 to establish the communication link 50 with the ground station 30. The ground station 30 can transmit data via the communication link 50 to the communication device 28, in communication with the controller 42 and the memory 40. The data 60 transferred between the UAV 10 and the ground station 30 can be completed via the communication link 50.

The ground system 30 can be configured to receive data 60 collected by the UAV 10. The communication link 50 can be used to transfer any collected data 60 or other information to the ground system 30. A destination server or computer 32 is also illustrated and can indirectly communicate via the ground system 30 and the UAV 10. The computer 32 can be located at or in communication with the ground system 30. Optionally, any collected data 60 on the UAV 10 can be communicated directly to the computer 32 via the communication device 28. It will be understood that such data or information may be securely communicated to the computer 32 as needed. The communication device 28 can execute a program for transmitting any collected data 60 to the computer 32. It is contemplated that such a process can be user initiated, implemented automatically by the communication device 28, or queried by the computer 32.

Once the collected data 60 is transferred or otherwise relayed to the computer 32, the computer 32 can analyze the collected data 60 and determine various characteristics of the soil from one or more locations. The computer 32 can include a suitable computer processor or computer program product comprising machine-readable media for carrying or having machine-executable instructions or data structures stored thereon. A display 34 can be operably coupled to the computer 32 and the computer 32 can be configured to provide an indication or data output 36 to the display 34 that is representative of the collected data 60 or some portion thereof including a processed portion of the collected data 60. By way of non-limiting example, an indication of output could be a value representative of a characteristic of the soil or could be an analysis of the value as compared to an acceptable range such as a predetermined range or even a historic range. In addition, the computer 32 could track and determine various output trends such as determining whether trends from a dataset of different locations at different time falls within an acceptable range.

The computer 32 can comprise executable instructions 37, a processor 38 and a memory 40 configured to store values representative of soil characteristics, GPS measurements, depth measurements and other data and to analyze data and trends associated with the data in a machine learning database. The computer 32 can monitor trends of values and can compare values in one location to another location or compare values with a predetermined or historical range.

In one example, the system 1 might be configured to test for the uniform application of a certain a fertilizer or herbicide. The system 1 might be programmed to send the UAV 10 to various locations on the land 20 and collect a soil sample at a specific depth. If the UAV 10 is equipped with the appropriate sensors 24, the UAV 10 can analyze the soil characteristics on board and store the data in the UAV memory 40. Otherwise, the UAV 10 can collect a soil sample from a given depth and fly the sample back to the ground station 30 for soil analysis by the one or more sensors 24. The sensors 24 can identify one or more values associated with a characteristic of the soil such a value indicative of the application of a fertilizer. The computer 32 can store the values of the characteristics of the soil determined by the sensors 24 and can compare the values from location to location to determine whether a fertilizer application has been applied uniformly. The computer 32 can determine locations where fertilizer has been over applied or under applied by comparing the values to an appropriate range such as a predetermined or historical range. If the computer 32 determines that a certain area has an under application of fertilizer, the computer 32 can alert the operator to apply additional fertilizer in the under application areas. If the computer 32 determines that a certain area has an over application of fertilizer, the computer 32 can alert the operator of the over application and the operator can take a corrective action for the next application. In such a way, the system 1 can help farmers or land developers optimize resources.

It should be recognized that the computer 32 could take other corrective actions as necessary depending on the soil sampling data. For example, a corrective action might involve delaying a scheduled application or scheduling an application to the soil at the location of treatment that would alter the value. In addition, if, for example, the computer 32 were connected to and able to control an automated fertilizer dispenser or an automated watering system, the computer could send instructions to either system to start or stop an application. Moreover, the computer could send instructions fertilize or water only portions of the farm land or to apply more or less water or fertilizer of various portions of the farm land.

In another example, the computer 32 can be separately connected to the Internet 70 for gathering information related to local weather forecasting. The local weather forecast can be downloaded into the computer 32 and machine learning database and further used for resource optimization. In some instances, fertilizers, herbicides and insecticides perform better in wet or dry conditions. Here, once the UAV 10 collects soil samples, the system 1 via the one or more sensors 24 can identify a value associated with soil moisture. If a certain fertilizer works better with a high moisture content, the system 1 can determine the current fertilizer and moisture level of the soil, identify that rain is in the forecast, and alert an operator to apply fertilizer if fertilizer levels are lower than a predetermined range and moisture content is high or expected to rise due to rain. Alternatively, if no rain is in the forecast, the computer 32 can send instructions to start an automated watering system.

In another example, some compliance with laws and restrictions on use of certain fertilizers and chemicals can be achieved. For example, the European Commission has fined countries and localities for an inability to control nitrate run off from farms. This occurs as a result of over application of fertilizer by farmers in a quest to improve yield. In this scenario, either the farmer or the government could use UAV's to monitor farm land to understand which lands are contributing to the nitrate overuse.

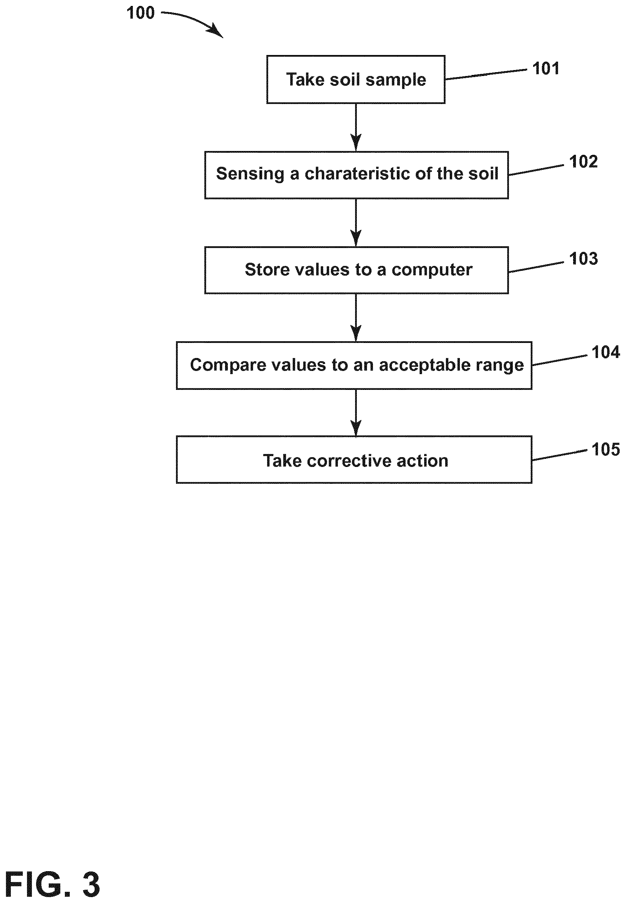

FIG. 3 is a flow chart illustrating a method 100 of determining soil condition for possible treatment. The method 100 comprises taking a soil sample at a predetermined location with unmanned aerial vehicle (UAV) at step 101. At this step, the UAV can be preprogrammed or manually controlled by a land owner to fly over a portion of the land. The UAV 10 can carry tools 24 capable of collecting a soil or vegetation sample at a specific depth. Once the soil sample is taken, the next step 102 is sensing at least one characteristic of the soil with a sensor. At this step, the UAV 10 may be equipped with a sensor 24 configured to sense soil characteristics. If not, the UAV 10 can be flown to a ground station that has a sensor capable of sensing soil characteristics. Once the soil characteristics are identified as values, the values are transmitted to a computer 32 where at step 103 the values representative of the soil characteristics and the location of the soil sampling collect are stored in the computer 32, such as in a database. At step 104, the computer 32 analyzes the values and determines if the values are within an acceptable range. One way of determining an acceptable ranges is comparing the ranges to predetermined or historical value ranges. If the values are not in an acceptable range, then at step 105, the computer 32 can take a corrective action to adjust the valve to move the value within the acceptable range. At this step, the computer 32 may send instructions to an automated fertilizer dispenser or an automated watering system to start or stop an application.

Aspects of the present disclosure provide for a variety of benefits including providing optimization of resources for farming and improving overall farm yields. Additionally, the present disclosure may be useful to farmers or government officials for ensuring compliance with local laws and regulations governing use of certain fertilizers, insecticides or herbicides.

To the extent not already described, the different features and structures of the various embodiments can be used in combination with each other as desired. That one feature is not illustrated in all of the embodiments is not meant to be construed that it cannot be, but is done for brevity of description. Thus, the various features of the different embodiments can be mixed and matched as desired to form new embodiments, whether or not the new embodiments are expressly described. All combinations or permutations of features described herein are covered by this disclosure.

This written description uses examples to disclose the invention, including the best mode, and also to enable any person skilled in the art to practice the invention, including making and using any devices or systems and performing any incorporated methods. The patentable scope of the invention is defined by the claims, and can include other examples that occur to those skilled in the art. Such other examples are intended to be within the scope of the claims if they have structural elements that do not differ from the literal language of the claims, or if they include equivalent structural elements with insubstantial differences from the literal languages of the claims.

Various aspects and embodiments of the present invention are defined by the following numbered clauses:
1. A method of determining soil condition for possible treatment, the method comprising:
   a) taking a soil sample at a location with an unmanned aerial vehicle (UAV);
   b) sensing at least one characteristic of the soil with a sensor;
   c) storing a value representative of the at least one characteristic and the location in a database;
   d) determining if the value is within an acceptable range; and
   e) taking a corrective action to adjust the valve if the value is not within the acceptable range.
2. The method of clause 1, further comprising repeating a-c at different locations to define a dataset of different locations.
3. The method of any preceding clause, further comprising repeating a-c at different times for the different locations to define a dataset of different locations at different times.
4. The method of any preceding clause, wherein determining if the value is within an acceptable range comprises determining a trend from the dataset of different locations at different times.
5. The method of any preceding clause, wherein determining if the value is within an acceptable range comprises determining if the trend is within an acceptable range.
6. The method of any preceding clause, further comprising repeating a-c at different times for the predetermined location.
7. The method of any preceding clause, wherein the talking a corrective action comprises one of delaying a scheduled application or scheduling an application to the soil at the location of treatment that would alter the value.
8. The method of any preceding clause, further comprising monitoring weather forecasts for conditions that alter the value and the delaying or scheduling of the application of the treatment is based on the monitored weather forecast.
9. The method of any preceding clause, further comprising the step of flying the soil sample to a ground-based sensor with the UAV.
10. The method of any preceding clause, further comprising the step of analyzing the soil sample with a sensor on the UAV.
11. The method of any preceding clause, wherein the soil sample is taken from a depth of at least 6 inches below ground level.
12. A method of determining soil condition for possible treatment, the method comprising:
   a) taking a soil sample at a preprogrammed GPS location with an unmanned aerial vehicle (UAV);
   b) sensing, with a sensor, a characteristic of a resource applied to the soil;
   c) storing a value representative of the characteristic and the preprogrammed GPS location in a database;
   d) determining if the value is within an acceptable range; and
   e) taking a corrective action to adjust the valve if the value is not within the acceptable range.
13. The method of any preceding clause, further comprising repeating a-c at different preprogrammed GPS locations to define a dataset of different locations.
14. The method of any preceding clause, further comprising repeating a-c at different times for the different preprogrammed GPS locations to define a dataset of different locations at different times.
15. The method of any preceding clause, wherein determining if the value is within an acceptable range comprises determining a trend from the dataset of different locations at different times.
16. The method of any preceding clause, wherein determining if the value is within an acceptable range comprises determining if the trend is within an acceptable range.
17. The method of any preceding clause, wherein the talking a corrective action comprises one of delaying a scheduled application or scheduling an application to the soil at the location of treatment that would alter the value.
18. The method of any preceding clause, further comprising monitoring weather forecasts for conditions that alter the value and the delaying or scheduling of the application of the treatment is based on the monitored weather forecast.
19. The method of any preceding clause, further comprising the step of flying the soil sample to a ground-based sensor with the UAV.
20. The method of any preceding clause, further comprising the step of analyzing the soil sample with a sensor on the UAV.
21. The method of any preceding clause, wherein the soil sample is taken from a depth of at least 6 inches below ground level.

## Claims

1. A method (100) of determining soil condition for possible treatment, the method (100) comprising:
a) taking a soil sample (101) at a location with an unmanned aerial vehicle (UAV) (10);
b) sensing (102) at least one characteristic of the soil with a sensor (26);
c) storing a value representative of the at least one characteristic (103) and the location in a database;
d) determining if the value is within an acceptable range (104); and
e) taking a corrective action (105) to adjust the valve if the value is not within the acceptable range.

2. The method of claim 1, further comprising repeating a-c at different locations to define a dataset of different locations.

3. The method of claim 2, further comprising repeating a-c at different times for the different locations to define a dataset of different locations at different times.

4. The method of claim 3, wherein determining if the value is within an acceptable range comprises determining a trend from the dataset of different locations at different times.

5. The method of claim 4, wherein determining if the value is within an acceptable range comprises determining if the trend is within an acceptable range.

6. The method of claim 1, further comprising repeating a-c at different times for the predetermined location.

7. The method of any preceding claim, wherein the talking a corrective action comprises one of delaying a scheduled application or scheduling an application to the soil at the location of treatment that would alter the value.

8. The method of claim 7, further comprising monitoring weather forecasts for conditions that alter the value and the delaying or scheduling of the application of the treatment is based on the monitored weather forecast.

9. The method of any preceding claim, further comprising the step of flying the soil sample to a ground-based sensor with the UAV (10).

10. The method of any preceding claim, further comprising the step of analyzing the soil sample with a sensor on the UAV (10).

11. The method of any preceding claim, wherein the soil sample is taken from a depth of at least 6 inches below ground level.
